**Europäisches Patentamt**

(19) **European Patent Office**

**Office européen des brevets**

(11) Publication number: **0 271 361**
**A2**

(12) # EUROPEAN PATENT APPLICATION

(21) Application number: **87310942.5**

(22) Date of filing: **11.12.87**

(51) Int. Cl.⁴: **H 01 H 7/00**

(30) Priority: **12.12.86 US 940803  26.05.87 US 54415**

(43) Date of publication of application:
**15.06.88 Bulletin 88/24**

(84) Designated Contracting States:
**BE CH DE FR GB IT LI LU NL**

(71) Applicant: **PIEZO ELECTRIC PRODUCTS, INC.**
**186 Massachusetts Avenue**
**Cambridge, Massachusetts 02139 (US)**

(72) Inventor: **Carter, Robert E.**
**21 Washburn Avenue**
**Auburndale Massachusetts 02166 (US)**

**Stone, W. Porter**
**1835 Union Street**
**Walpole New Hampshire 03608 (US)**

(74) Representative: **Cross, Rupert Edward Blount et al**
**BOULT, WADE & TENNANT 27 Furnival Street**
**London EC4A 1PQ (GB)**

(54) **Delayed actuator.**

㊼ A visco-elastic actuator includes a support section (12) and an actuator section (28). A visco-elastic adhesive (24) serves to hold the actuator in a first state. A spring (40) acts between the sections until the hold of the adhesive (24) is overcome when the actuator is switched to a second state.

*FIG. 1*

EP 0 271 361 A2

**Description**

DELAYED ACTUATOR

This invention relates to a delayed actuator, that is an actuator as used to operate devices such as relays, valves and fluid dispensers, by temporarily holding such devices in a first state and then abruptly and automatically switching them to a second state.

Time delayed actuators are widely employed to switch relay contacts, open and close fluid control valves and operate many other types of mechanism. Electrical and electronic timers are perhaps the most commonly used actuators of this type. In certain applications, however, such timers are impractical because of their expense, complexity and/or need for a power supply.

As an alternative to the electrical timer a dashpot type of delayed actuator may be utilized. Such an actuator includes a piston disposed within a fluid filled cylinder. As the piston is moved through the cylinder fluid is forced through a circumferential gap between the piston and the cylinder wall so that the piston gradually changes from a first state to a second state.

Another type of actuator is a rotary plate delayed actuator as used to slow the motion of cassette tape machine doors and record player armatures. Such an actuator employs a pair of parallel plates that are separated by a layer of viscous fluid. Torque is applied to one of the plates while the other is held fixed and the viscous change of the fluid slows the motion of the movable plate.

Typically, dashpot and rotary plate actuators are effective for providing delays of only a few seconds. In order to extend their delay periods their fluid reservoirs must be made impractically large. Increasing the delay of the dashpot activator alternatively requires making the circumferential gap exceedingly and impractically small. Moreover, both types of actuator must be hermetically sealed to prevent escape or contamination of the fluid, and each requires at least one precision tolerance, i.e. the dashpot piston/cylinder clearance or the rotary plate spacing, which adds significantly to the cost of the actuator.

There is a particular need for an inexpensive and effective time delayed actuator for dispensing fragrances, insecticides and other airborne fluids only when needed. Present dispensers are either passive or active. Passive dispensers employ a container filled with a fluid. A wick protruding from the fluid filled container absorbs the fluid and emits it into the air. These dispensers do not employ extremely volatile fluids because such fluids evaporate too rapidly. However, as a result, the emission rate of these dispensers is often not sufficient to perform the function desired, e.g., the elimination of offensive odours.

Less volatile fluids may be dispensed more effectively with an active dispenser that employs an electric blower or heater to stimulate emission. However, such dispensers are fairly expensive and again require a source of electrical power.

According to this invention there is provided a delayed actuator characterised by a support section; an actuator section; visco-elastic adhesive means operative to hold the support section and the actuator section in a first state relative to each other; and resilient means operative when the support section and actuator section are in said first state to act against the hold of the adhesive means until the adhesive hold is broken whereupon the support and actuator sections adopt a second state relative to each other.

The invention provides a delayed actuator which is relatively uncomplicated and simple to manufacture and operate, and which does not require a source of electricity, precision parts or a hermetic seal.

The actuator of the invention can remain in a first state for an extended period of time, and then rapidly, reliably and automatically switch to a second state.

Further, the actuator of the invention can be used for operating a wide variety of fluid dispensers, valves, relays and other mechanisms, and will enable a fluid dispenser to dispense effective amounts of fluid only as needed.

The adhesive means can act directly between the support section and the actuator section.

Otherwise the actuator can include restraining means operative to hold the actuator section and the support section in said first state and released to allow the support section and the actuator section to adopt said second state when the hold of the adhesive means is broken by the resilient means.

Such an actuator can provide prolonged time delays for large and/or heavy mechanisms.

The use of the adhesive means simply as a timer controlling a separate retaining means gives a holding force that is not dependent on the strength of the adhesive means.

This invention will now be described by way of example with reference to the drawings, in which :-

Fig. 1 is an elevational cross-sectional view of a fluid dispenser using a visco-elastic delayed actuator according to this invention;

Fig. 2 is an elevational cross-sectional view of a visco-elastic delayed actuator according to this invention, used as a relay, in a first state with its contacts separated;

Fig. 3 is a view similar to that of Fig. 2 but showing the relay closing;

Fig. 4 is a view similar to that of Figs. 2 and 3 but showing the relay in a second state with its contacts engaged;

Fig. 5 is an elevational cross-sectional view of another visco-elastic delayed actuator relay according to this invention;

Fig. 6 is an elevational cross-sectional view of a further visco-elastic delayed actuator according to this invention for operating a fluid control valve;

Fig. 7 is a simplified plan view of another visco-elastic actuator according to this invention used as a relay;

Fig. 8 is a perspective view of a fluid dispenser utilizing a delayed actuator according to this invention;

Fig. 9 is a cross-sectional view of the fluid dispenser of Fig. 8 taken along line IX-IX of Fig. 8 with the actuator in a first state;

Fig. 10 is a perspective view of a restraining means of Fig. 9.

Fig. 11 is a view similar to that of Fig. 9 but with the actuator in a second state;

Fig. 12 is a perspective view of another fluid dispenser using a delayed actuator according to this invention;

Fig. 13 is a cross-sectional view taken along line XII-XII of Fig. 12;

Fig. 14 is an elevational view of another delayed actuator according to this invention; and Fig. 15 is an elevational view taken along line XV-XV of Fig. 14.

The actuators to be described utilise a visco-elastic adhesive such as STICK EM (trade mark) brand mouse trap glue manufactured by J T Eaton and Company, to hold an actuator section and a support section in a first state. Resilient means act against the adhesive hold until it is abruptly broken to switch the support section and the actuator section into a second state.

The desire time delay is provided while the actuator and support sections are held in the first state by the visco-elastic adhesive. Accordingly, the visco-elastic adhesive typically must exhibit a high surface adhesion or stickiness. By selecting a visco-elastic adhesive with suitable adhesion, time delays of minutes to hours and even days may be provided.

By selecting a stickier visco-elastic adhesive a longer delay time is provided and conversely by utilizing a less viscous or less sticky adhesive a shorter delay time is achieved. For example, where an adhesive having a viscosity of $10342.5 N_s/m^2$ (0.4 ounce min/in$^2$ is utilized on a $11.5 cm^2$ (2 in$^2$) surface and a 25 gram force is applied, a delay of twenty minutes is achieved. Applying the same force but using an adhesive with a viscosity of $1034.25 N_s/m^2$ (0.04 ounce min/in$^2$) yields a delay of five minutes.

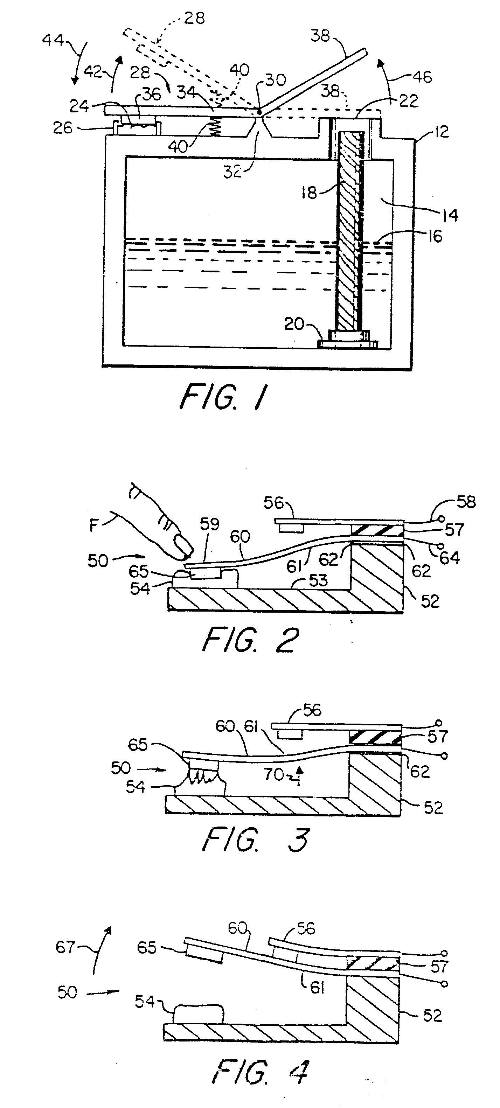

There is shown in Fig. 1 a fluid dispenser 10 which incorporates a delayed actuator comprising a support section 12 in the form of a housing with an interior chamber 14. The chamber accommodates a fluid 16 which may be a fragrance, insecticide or other volatile substance to be dispensed. A wick 18 is mounted in holder 20 within chamber 14 and extends into opening 22 of housing 12. Wick 18 absorbs volatile fluid 16 and, with opening 22 uncovered, dispenses the fluid as a vapor through opening 22 and into the surrounding environment.

A visco-elastic adhesive 24 is held in a container 26 which is mounted on the upper surface of housing 12. An actuator 28 is pivotably mounted by pivot 30 to fulcrum 32 of housing 12.

The actuator includes a lever portion 34 that carries an engagement member 36 at one end. a closure portion 38 is integrally attached to the opposite end of lever portion 34. A compression spring 40 acts between housing 12 and actuator 28 to urge actuator 28 to pivot upwardly in the direction of arrow 42.

In order to dispense fluid for a desired period of time actuator 28 is pivotably lowered, either manually or automatically by means not shown, in the direction of arrow 44 until engagement portion 36 intimately engages visco-elastic adhesive 24. This compresses spring 40 and raises closure portion 38 in the direction of arrow 46 to uncover opening 22. As a result, volatile fluid 16 is emitted as a vapour by wick 18. This emission continues for as long as engagement portion 36 of actuator 28 remains adhered to adhesive 24 and closure 38 remains open.

With actuator 28 in the open state and member 36 engaged with adhesive 24, compressed spring 40 urges the actuator apart from housing 12, e.g., in the direction of arrow 42. Gradually the engagement portion 36 of actuator 28 is pulled apart from adhesive 24. Finally, the spring force of compression spring 40 overcomes the holding force of adhesive 24 and actuator 28 breaks suddenly away from the adhesive and moves rapidly in the direction of arrow 42 to the state shown in phantom so that closure portion 38 is pivoted downwardly to cover opening 22. As a result, no further fluid is dispensed through the opening.

A visco-elastic delayed relay 50 is shown in Figs. 2-4. The relay includes a support section 52 which has an adhesive supporting portion 53 for supporting a visco-elastic adhesive 54. A first contact 56 is mounted to the upper surface of raised portion 57 of support section 52 and is connected via an electrode 58 to an electrical circuit not shown. The relay 50 includes an actuator section 59 comprising a second electrical contact 60 formed from a metal leaf spring 61 which is received in an opening 62 in support section 52. Contact 60 is similarly connected via an electrode 64 to the electrical circuit.

Leaf spring 61 is biased to urge contact 60 upwardly into engagement with contact 56. In order to temporarily separate contacts 56 and 60 and open the electrical circuit, actuator section 59 is urged downwardly apart from contact 56 such as by the operator's finger F, Fig. 2. Leaf spring 61 is bent and engagement portion 65 of actuator 59 is urged into intimate contact with adhesive 54. Then, for as long as engagement portions 65 remains adhered to adhesive 54, contacts 56 and 60 remain separated and the circuit remains open.

As time passes, Fig. 3 spring 61 urges actuator section 59 apart from adhesive supporting portion 53 of support section 52. In particular, engagement portion 65 is caused to gradually separate from adhesive 54. This separation may require minutes or even hours, depending upon the level of adhesion or stickiness of adhesive 54. However, for as long as at least some adhesion remains, contacts 56 and 60 continue to be separated.

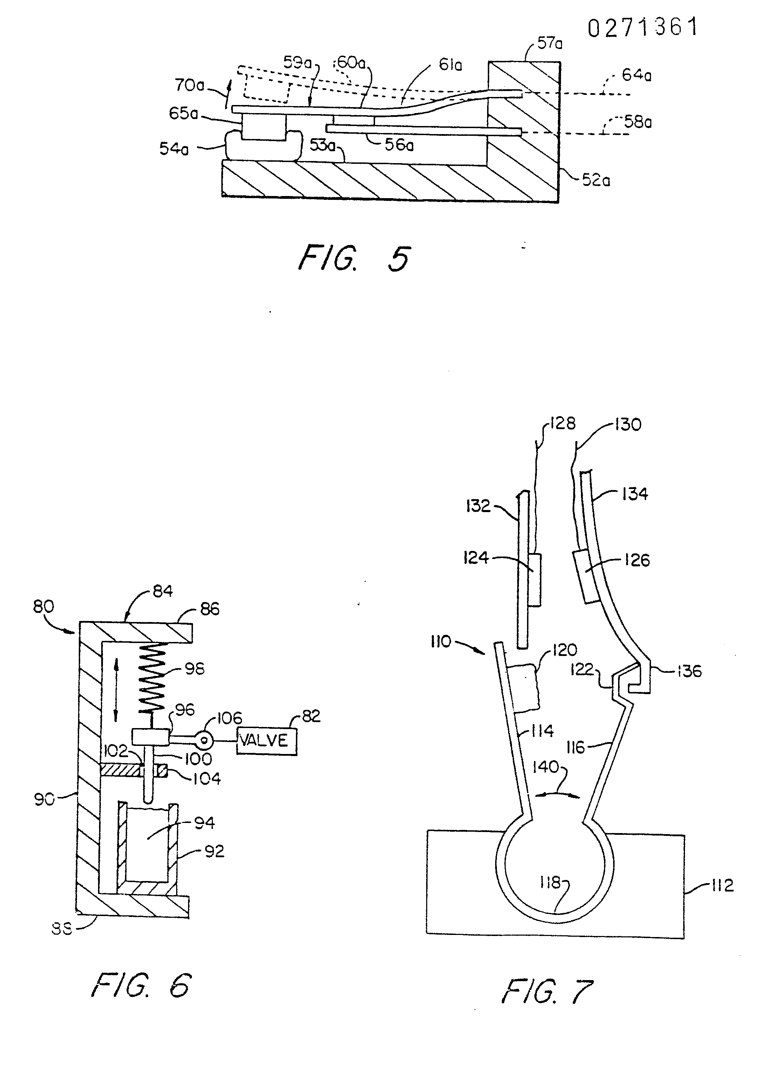

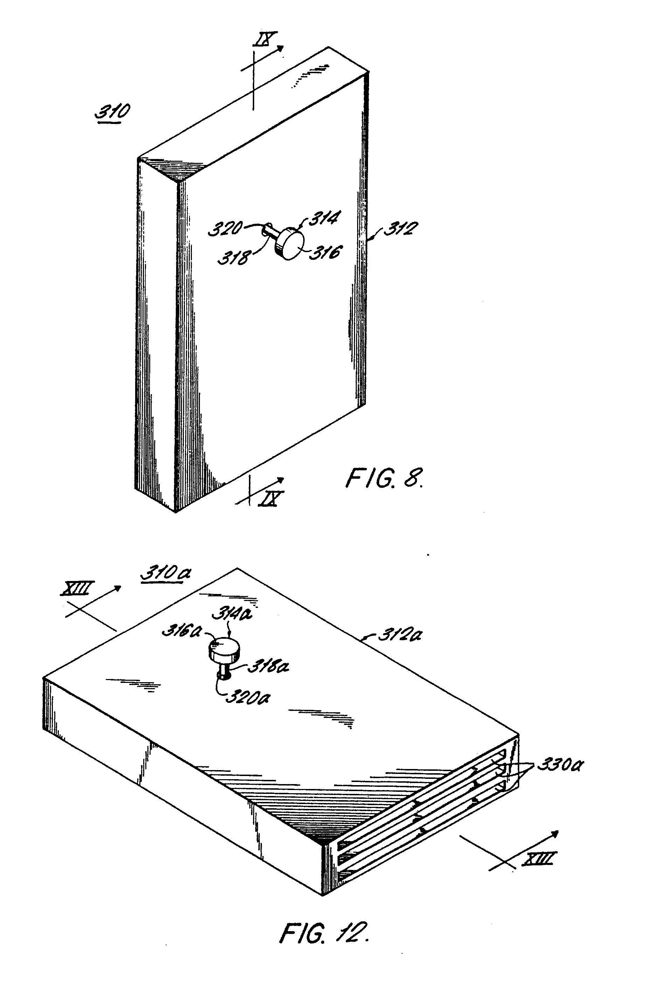

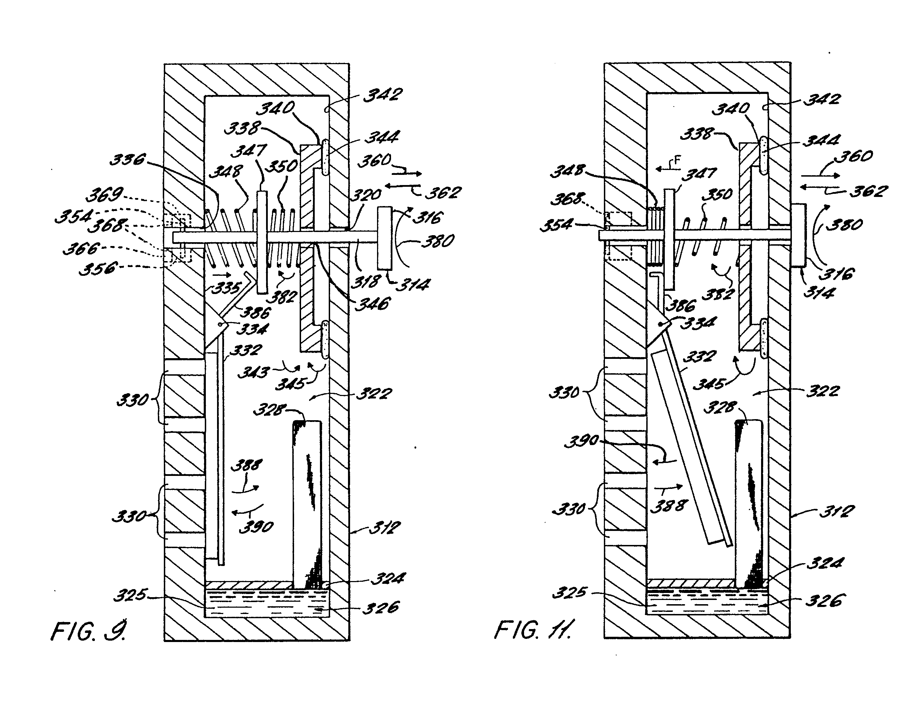

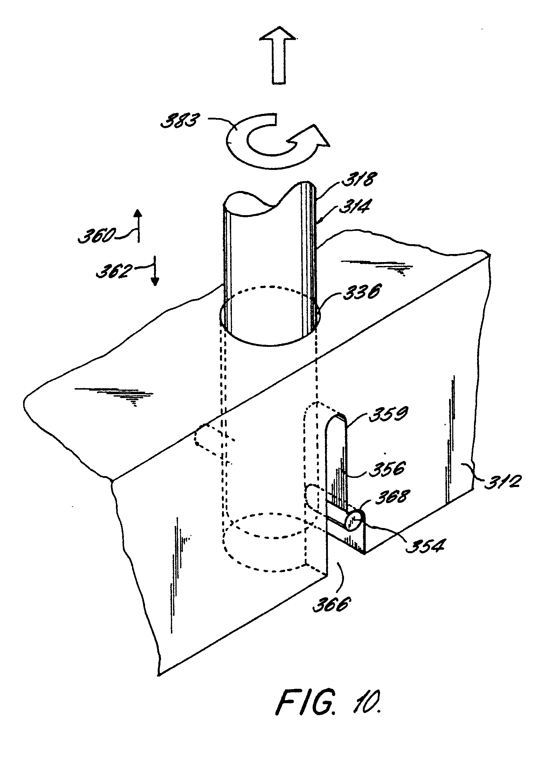

Finally, the force of spring 61 overcomes the adhesive restraint of visco-elastic adhesive 54 and, as shown in Fig. 4, engagement member 65 breaks suddenly away from adhesive 54 in the direction of arrow 67. This causes contact 60 to engage contact 56 and close the electrical circuit.

In an alternative embodiment, Fig. 5, stationary contact 56a is mounted on support section 52a between adhesive supporting portion 53a and the actuator section 59a carrying contact 60a. Again, the actuator includes an integral metal leaf spring 61a which is attached to raised support portion 57a, forms a contact 60a and carries an engagement portion 65a at is distal end. Spring 61a urges actuator section 59a apart from adhesive 54a mounted on support section 52a.

By urging actuator section 59a downwardly so that engagement portion 65a intimately engages adhesive 54a, contact 60a makes electrical contact with contact 56a. As a result, the electrical circuit, not shown, which is connected to contact 60a and 56a by electrodes 64a and 58a, respectively is closed. The circuit remains closed and operating for the duration of the timing cycle, i.e., for as long as engagement portion 65a remains engaged with adhesive 54a. Spring 61 continues to urge actuator section 59 upwardly in the direction of arrow 70a. For the majority of the timing cycle, e.g., 90% or more, engagement portion 65a remains adhered to adhesive 54a. Eventually, however, the force of leaf spring 61a overcomes the adhesive engagement and actuator section 59a is snapped suddenly apart from adhesive 54a in the direction of arrow 70a to the psotioin shown in phantom. This separates contact 60a from contact 56a and opens the circuit.

As shown in Fig. 6, an actuator 80 according to this invention may be employed to open and close a valve 82. Actuator 80 includes a support section 84 having an upper portion 86, a lower portion 88 and an intermediate portion 90 which interconnects portions 86 and 88. A container 92 filled with visco-elastic adhesive 94 is mounted on lower support portion 88. An actuator section 86 is suspended from upper support portion 86 by a helical spring 98. Actuator section 96 includes an insertion member 100 which extends through an opening 102 in a guide 104 that is mounted to intermediate section 90 of support 84. An arm 106 extending from actuator section 96 operates valve 82 in a conventional manner not shown.

In operation, actuator section 96 is lowered so that helical spring 98 is extended and insertion member 100 passes through guide 104 and into adhesive 94. Insertion member 100 temporarily adheres to adhesive 94 so that valve 82 is closed. At the same time, extended spring 98 urges actuator section 96 upwardly so that insertion member 100 is gradually pulled out of adhesive 94. Eventually, near the end of the timing cycle the force of spring 98 pulls insertion member 100 completely and suddenly out of adhesive 94. Adhesive engagement is suddenly broken and arm 106 quickly switches valve 82 to an open condition.

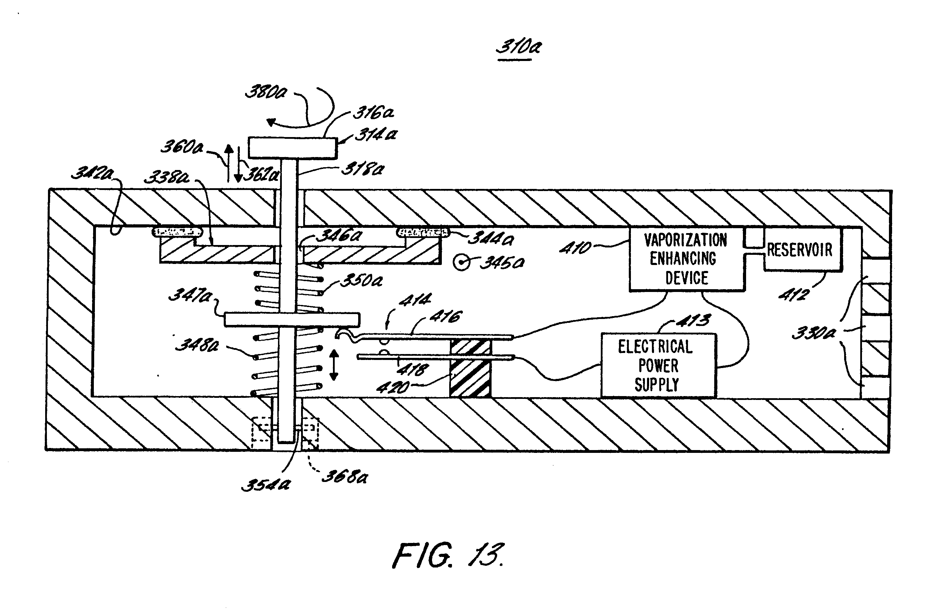

A one-piece timed delay actuator 110 is shown in Fig. 7. Actuator 110 is mounted in a base 112 and it comprises a unitary leaf spring including a support section 114, an actuator section 116 and a resilient junction section 118 which joins sections 114 and 116 and urges sections 114 and 116 apart. A visco-elastic adhesive 120 is mounted proximate the distal end of support section 114. The distal end of actuator section 116 forms a hooked portion 122.

Actuator 110 is used to open and close a pair of electrical contacts 124 and 126 which are connected to an electrical circuit, not shown, through respective electrodes 128 and 130. Contacts 124 and 126 are mounted on respective switch members 132 and 134 which are biased by means not shown to urge the contacts together. Member 134 includes a hooked portion 136 which engages hook portion 122 of actuator 116. With sections 114 and 116 in the separated state, hook portion 122 holds switch member 134 apart from member 132 and thereby separates contacts 124 and 126. As sections 114 and 116 are urged together the holding force of hook portion 122 is removed and separation between switch members 132 and 134 and the contacts 124 and 126 that they carry is reduced until the contacts engage. The bottom of hook portion 122 intimately engages adhesive 120 and actuator section 116 is held temporarily in a state engaged with section 114 so that contacts 124 and 126 remain closed. During this timing cycle spring 118 urges sections 114 and 116 apart in the direction of double-headed arrow 140. Gradually, the hooked portion 122 separates from adhesive 120. Finally, the force of spring 118 totally overcomes the adhesive retaining force and hook portion 122 snaps suddenly apart from adhesive 120. As a result, hook portion 122 deflects switch member 134 and again separates contacts 124 and 126.

Although in each of the actuators described above the actuator section is mounted to the support section, this is not essential and in other actuators the actuator and support sections may be mounted entirely independently of one another.

With the actuators described above the adhesive serves as timing means and also as a retaining means serving to hold the actuator in the first state, that is to hold the support and actuator sections together against the action of the resilient means.

However, other arrangements are possible, as will now be described, and in particular arrangements in which the adhesive serves only as a timing means controlling a separate retaining means serving to hold the actuator in the first state.

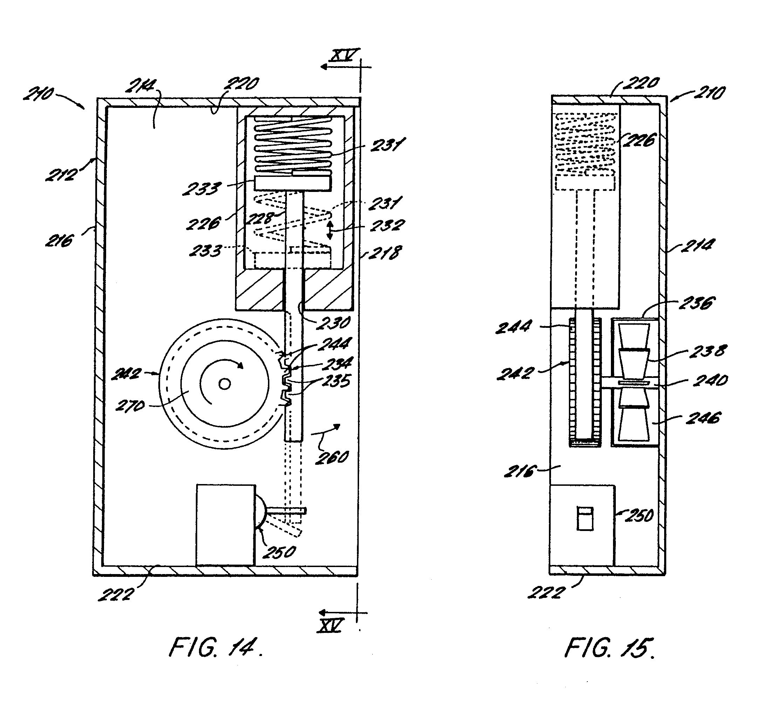

Referring now to Fig. 8, this shows a delayed fluid dispenser 310 including a support section housing 312 which has fluid dispensing ports on the side not shown. An actuator section 314 is rotatably and slidably mounted within housing 312 and includes an external knob 316 and a shaft 318 which extends into housing 312 through opening 320.

The internal construction of dispenser 310 is shown in detail in Fig. 9. Housing 312 includes an inner chamber 322 within which is disposed a reservoir 326 of a fragrance, insecticide or other volatile fluid 325. An absorbent wick 328 is mounted within chamber 322 with one end disposed through plate 324 into fluid 325. Wick 328 draws fluid from reservoir 326, which fluid is subsequently emitted by the wick as a vapor. To provide adequate emission, wick 328 should have an exposed surface area of several square centimetres. Fluid vapors are dispensed from dispenser 310 through ports 330 when the ports are opened to the outside air. Ports 330 are opened and closed by a closure 332 which is mounted by a pivot 334 to chamber wall 335 of housing 312 and is operated in a manner described more fully below.

Actuator shaft 318 externds through opening 320 and into opening 336 of housing 312. A movable member in the form of a circular rotor 338 includes a circumferential bearing surface 340 which is connected to chamber wall 342 by a visco-elastic adhesive 344 which permits rotor plate 338 to rotate relative to chamber wall 342 in the direction of arrows 343 and 345 while remaining attached to the chamber wall. Rotor 338 also includes a central hole 346 through which actuator shaft 318 extends.

A circular engagement plate 347 attached to actuator shaft 318 approximately mid-way along the length of the shaft. Engagement plate 347 may be integral with shaft 318 or, alternatively, may be attached thereto by suitable adhesive, bolts or other attachment means. A helical compression spring 348 is wound about shaft 318 and is attached at one end to chamber wall 335 and at the other end to a first side of plate 347. A helical torsion spring 350 is likewise wound about shaft 318 and is attached at one end to the other side of plate 347 at the other end to rotor 338.

As shown most clearly in Fig. 10, a retaining detent 354 is mounted through the lower end of shaft 318. Housing 312 includes a recess 356 which receives detent 354 and permits actuator 314 to slide back and forth within housing 312 in the direction of arrows 360 and 362, Figs. 9 and 10. Recess 356, Fig. 10, includes an enlarged portion 366 proximate the outside of housing 312 which forms a shoulder 368 that engages and holds detent 354 when actuator 314 is retracted in the direction of arrow 362.

During periods when fluid dispensing is not required, the biasing force of compression spring 348, Fig. 9, urges plate 347 and actuator 314 in the direction of arrow 360 to remain in an extended condition. In this state detent 354 is received within narrow section 369 of slot 356 and does not engage shoulder 368. As a result, engagement plate 347 does not exert leverage on lever arm 386 and either gravity or a spring not shown biases closure 332 into a closed condition covering ports 330 and blocking emission of fluid 325.

In order to dispense fluid the operator grasps knob 316, rotates it clockwise in the direction of arrow 380 and depresses actuator 314 in the direction of arrow 362 into the position shown in Fig. 11. This operation winds spring 350 in the direction of arrow 382 andcompresses spring 348. Wound spring 350 exerts a torque in the direction of arrow 382 which engages detent 354 with shoulder 368, as shown in Figs. 10 and 11. As actuator 314 is depressed, plate 347 engages lever arm 386 of closure 332 and causes the closure to pivot open in the direction of arrow 388. Ports 330 are thereby uncovered and fluid from wick 328 is emitted through the ports. The ports remain open and emission continues for as long as pin 354 is restrained by detent 368 and actuator 314 remains in the depressed condition of Fig. 11.

Closure 332 covers the ports after a timed duration as follows.

In the retracted condition of Fig. 11 torsion spring 350 is wound relatively tightly. As a result, the torque 383 it exerts on actuator shaft 318 keeps detent 354 engaged with shoulder 368. As long as sufficient torque is exerted spring 348 remains compressed and actuator 14 remains in the depressed condition. Wound torsion spring 350 also exerts a torque 382 on rotor 338 which causes the rotor to tend to rotate relative to chamber wall 342 in the direction of arrow 345. This rotation is hindered and prolonged by visco-elastic adhesive 344 which joins rotor 338 and chamber wall 342. Eventually, after a time which is dependent upon the viscosity and stickiness of adhesive 344, rotor 338 rotates sufficiently in the direction of arrow 341 to unwind torsion spring 350. As a result, torque 383 is removed from actuator 314, detent 354 disengages from shoulder 368 and the biasing force of spring 348 suddenly overcomes the restraining force and snaps actuator 314 in the direction of arrow 360 into the extended condition of Fig. 9. As a result, the leverage exerted by plate 347 on lever arm 386 is removed and closure 332 pivots downwardly in the direction of arrow 390 to reclose ports 330 and block fluid emission.

The time delay in the open state and hence the period of fluid emission may be increased by employing a stickier adhesive 344 which slows the movement of rotor 338 and unwinding of spring 350. Conversely, the time delay may be shortened by using a less viscous or sticky adhesive.

An alternative dispenser 310a is shown in Fig. 5, which dispenser includes a housing 312a and an actuator 314a which extends into the housing through an opening 320a. Actuator 314a includes an external knob and an elongate shaft 318a. A plurality of elongate ports 330a are disposed in one end of the housing.

As shown in Fig. 13, dispenser 310a includes an actuator 314a, compression spring 348a, restaining detent 354a, shoulder 368a, torsion spring 350a, engagement plate 347a, rotor 338a and visco-elastic adhesive 344a which are constructed and operate identically to the components shown in the arrangement of Figs. 1 to 4. Instead of a mechanical closure, however, dispenser 310a is provided with an electrically operated vaporization enhancing device 410 which when activated increases vaporization of fluid from reservoir 412. Device 410 may include, for example, a fan which utilizes motor-driven properllors or oscillating blades, an electrical resistance heater or an ultrasonic atomizer.

An AC or DC power source 413 is connected to device 410 through a switch 414 including a pair of normally open contacts 417 and 418 which are mounted on a support 420 within housing 312a.

With actuator or 314a in the extended condition, compression spring 348a raises engagement plate 347a sufficiently so that contacts 416 and 418 remain open. As a result, vaporization enhancing device 410 remains shut off and no fluid is emitted through ports 330a.

To operate dispenser 310 for a timed duration knob 316a is grasped and rotated in the direction of arrow 380a and actuator 314a is depressed in the direction of arrow 362a so that detent 354a is engaged with shoulder 368a in the manner shown in Fig. 10. This winds torsion spring 350a to generate a torque on actuator 314a which engages detent 354a with shoulder 368a. As a result, actuator 314a and plate 347a are maintained in a depressed condition so that engagement plate 347a holds contacts 415 and 418 closed. This completes the electrical circuit and operates device 418 to enhance vaporization of fluid in reservoir 412. The vaporized fluid is then emitted through ports 330a.

The actuator swiches back to the extended condition after the desired time delay in the manner similar to the previously described arrangement of Figs. 1 to 4. Torsion spring 350a urges rotor 338a to rotate relateive to inner chamber wall 342a in the direction of arrow 345a. Due to visco-elastic adhesive 344a this rotation is very gradual. Eventually, spring 350a is unwound or relaxed by the rotation of rotor 338a and provides no torque to hold detent 354a in engagement with shoulder 368a. As a result, spring 348a suddenly snaps actuator 314a in the direction of arrow 360a back into the extended condition. Plate 347a is raised and contacts 416 and 418 again separate to open switch 414 and shut off vaporization enhancing device 410.

Another actuator 210 according to this invention is shown in Figs. 14 and 15, which includes a support section 212 which is open in the front and includes a rear wall 214, side walls 216 and 218 and top and bottom walls 220, 222.

A guide 226 is attached to walls 218 and 220 such as by a suitable adhesive. an actuator 228 extends thorugh a slot 230 in guide 226 and is slidable up and down in the directions of double-headed arrow 232. Actuator 228 carries a rack 234 below guide 226. A helical compression spring 231 is attached at one end to guide 226. The opposite end of spring 231 engages a knob 233 at the top of actuator 228. As shown in phantom, the compression spring urges actuator 228 into an extended condition so that know 233 engages guide 226 adjacent to slot 230.

A compartment 236, Fig. 15, is attached by screws, bolts, adhesive or similar attachment means to wall 214 of support section 212. A paddle wheel device 238 is rotatably mounted by a shaft 240 to wall 214. Shaft 240 extends through compartment 236 and is attached axially to a pinion gear 242 having teeth 244. A visco-elastic adhesive 246 fills compartment 236 and, interconnects the compartment with paddle wheel device 238.

A toggle switch 250 is mounted on bottom wall 222 of support section 212. Switch 250 includes resilient means, not shown, which urge the switch upwardly into an "on" condition. However, when the actuator is extended, as shown in phantom, switch 250 is depressed so that it switches into an alternative condition, e.g., an off state.

To operate timer 210, knob 233 is grasped and actuator 228 is retracted upwardly against spring 231. The actuator is composed of a flexible material so that it bends slightly in the direction of arrow 260. This enables rack teeth 235 to slide over pinion teeth 244. As a result, actuator 228 may be raised without having to rotate pinion gear 242 and paddle wheel device 238 against the resistance of viscous material 246 in compartment 236. In the retracted condition shown in Fig. 1, actuator 228 is spaced from toggle switch 250 and the switch maintains an on state. During the timed duration spring 231 urges actuator 228 downwardly so that rack teeth 235 engage and mesh with pinion teeth 244. This drives gear 242 in the direction of arrow 270 so that paddle wheel 238 turns within compartment 236. Visco-elastic adhesive 246 slows the rotation of paddle wheel 238 and pinion gear 242. Accordingly, the movement of rack 235 and actuator 228 in a downward direction is similarly delayed. The degree of this delay is determined by the viscosity of adhesive 246. A more viscous adhesive will cause slower movement of actuator 228 and, conversely, a less viscous adhesive will enable the actuator to extend more rapidly from guide 226.

Eventually, actuator 228 moves enough so that rack 234 extends beyond pinion gear 242; hence, rack teeth 235 disengage pinion teeth 244 and the rack and pinion no longer restrain or retard extension of actuator 228. The actuator abruptly switches to the fully extended position shown in phantom and as a result the bottom end of actuator 228 engages and depresses switch 250 and abruptly changes the switch to an "off" condition.

**Claims**

1. A delayed actuator characterised by a support section (12; 52; 84; 114; 312; 212); an actuator section (28; 59; 96; 116; 314; 228); visco-elastic adhesive means (24; 54; 94; 120; 44; 246) operative to hold the support section and the actuator section in a first state relative to each other; and resilient means (40; 61; 98; 118; 350; 231) operative when the support section and actuator section are in said first state to act against the hold of the adhesive means until the adhesive hold is broken whereupon the support and actuator sections adopt a second state relative to each other.

2. An actuator as claimed in Claim 1, characterised in that said support section (12; 312) includes a chamber (14; 26) for containing a fluid (16; 25) and an opening (22; 30) for dispensing said fluid.

3. An actuator as claimed in Claim 2, characterised in that said actuator section includes a closure section (38; 332) for alternatively covering and uncovering said opening (22; 30) to respectively block and permit the dispensing of said fluid.

4. An actuator as claimed in Claim 3, characterised in that said closure section includes a lever portion(34; 386) pivotably mounted to said support section (12; 312) and a closure portion (38; 332) connected to said lever portion (34; 386) for covering and uncovering said opening (22; 30).

5. An actuator as claimed in Claim 1, characterised by first electrical contact means (56) mounted on said support section (52), said actuator section (59) including second electrical contact means (60) for making selective electrical contact with said first electrical contact means (56) in one of said first and second states and being spaced from said first electrical contact means (56) in the other of said first and second states.

6. An actuator as claimed in Claim 5, characterised in that said support section (52) includes an adhesive supporting portion (53), said second electrical contact means (60) being mounted between said first electrical contact means (56) and said adhesive supporting portion (53) for engaging said first electrical contact means (56) when said support section and said actuator section (59) are in said second state.

7. An actuator as claimed in Claim 5, characterised in that said support section (52a) includes an adhesive supporting portion (53a) said first electrical contact means (56a) being carried between said second electrical contact means (60a) and said adhesive supporting portion (53a) for engaging said first electrical contact means (56a) when said actuator section (59a) is in the first state.

8. An actuator as claimed in any one of Claims 5 to 7, characterised in that said resilient means comprises a leaf spring (61; 61a) carrying said second contact means (60; 60a).

9. An actuator as claimed in claim 1, characterised in that said actuator section (96) includes an insertion member (100) which is selectively insertable in said adhesive means (94).

10. An actuator as claimed in Claim 9, characterised in that said support section (84) includes a lower support portion (88) for supporting said adhesive means (94), an upper support portion (86) and means (90) interconnecting said upper and lower portions (86, 88), said actuator section (96) being suspended by said resilient means (98) from said upper support portion (86).

11. An actuator as claimed in Claim 1, characterised in that said support section (114), resilient means (118) and actuator section (116) comprise a unitary leaf spring.

12. An actuator as claimed in any preceding claim, characterised in that said adhesive means acts directly between the support section and the actuator section.

13. An actuator as claimed in any one of Claims 1 to 4, characterised by restraining means (354; 356) operative to hold the actuator section and the support section in said first state and released to allow the support section and the actuator section to adopt said second state when the hold of the adhesive means is broken by the resilient means.

14. An actuator as claimed in Claim 13, characterised by a movable member (338) coupled to the support section (312) by the adhesive means (344) and coupled to the actuator section (314) by the resilient means (350).

15. An actuator claimed in Claim 14, characterised in that said restraining means includes a shoulder (368) disposed in said support section (312) and detent means (354) carried by said actuator section (314) for engaging said shoulder to hold the support section and the actuator section in said first state.

16. An actuator as claimed in Claim 15, characterised in that said movable member comprises a rotor (338), said resilient means comprises torque biasing means (350) responsive to the support section and the actuator section being placed in said first state to apply torque to urge said rotor to rotate relative to said support section against the hold of the adhesive means until said detent is disengaged from said shoulder and the support section and the actuator section are urged into said section state.

17. An actuator as claimed in Claim 16, characterised in that said torque biasing means comprises a helical torsion spring (350), the actuator section being axially slidably mounted in the support section, and being axially rotatable to wind said helical torsion spring as the support section and the actuator section are placed in said first state.

18. An actuator as claimed in any one of Claims 13 to 17, as dependent upon Claim 2, characterised by means (410) for accelerating dispersion of said fluid from said chamber.

19. An actuator as claimed in Claim 1, characterised by a movable member (438) coupled to the support section (414) by the adhesive means (446); first gear means (442) operably connected to said movable member (438) and engaged with complementary second gear means (434) connected to said actuator section and responsive to said resilient means (431) to drive said first gear means until said second gear means disengages from said first gear means and the support section and the actuator section adopt said second state.

20. An actuator as claimed in Claim 19, characterised in that said first gear means includes a rotatable pinion gear (442) and said second grear means includes rack means (434) arranged longitudinally on said actuator section (428).

21. An actuator as claimed in Claim 19 or Claim 20, characterised in that the support section includes compartment means (436) containing the adhesive means (446), said movable member being mounted in said compartment means.

22. An actuator claimed in Claim 21, characterised in that said movable member comprises a paddle wheel.

10

FIG. 1

FIG. 2

FIG. 3

FIG. 4

0271361

FIG. 5

FIG. 6

FIG. 7

FIG. 8.

FIG. 12.

FIG. 9.

FIG. 11.

0271361

FIG. 10.

*310a*

VAPORIZATION ENHANCING DEVICE

RESERVOIR

ELECTRICAL POWER SUPPLY

FIG. 13.

FIG. 14.

FIG. 15.

0271361